# EUROPEAN PATENT SPECIFICATION

(11) **EP 2 538 879 B1**
(45) Date of publication and mention of the grant of the patent: **08.04.2015**
(21) Application number: 11707012.8
(22) Date of filing: 23.02.2011
(51) Int. Cl.: A61F 2/24, A61B 5/107

(54) **CATHETER-BASED HEART VALVE THERAPY SYSTEM WITH SIZING BALLOON**
KATHETERBASIERTES HERZKLAPPENTHERAPIESYSTEM MIT GRÖSSENBESTIMMUNGSBALLON
SYSTÈME DE THÉRAPIE DES VALVULES CARDIAQUES BASÉ SUR UN CATHÉTER AVEC UN BALLONNET DE DIMENSIONNEMENT

(30) Priority: 23.02.2010 US 710852
(43) Date of publication of application: 02.01.2013
(73) Proprietor: Medtronic Inc., Santa Rosa, California 95403 (US)
(72) Inventor: TABOR, Charles, Minneapolis, Minnesota 55432 (US)
(74) Representative: Zimmermann & Partner
(86) International application number: PCT/US2011/025823
(87) International publication number: WO 2011/106354

(56) References cited:
- WO-A1-2005/084595
- WO-A2-02/094132
- WO-A2-2008/011261
- US-A1- 2004 093 060
- US-A1- 2008 009 746

## Description

### Background

The present disclosure relates to systems and methods for percutaneous implantation of a heart valve prosthesis. More particularly, it relates to systems and methods for transcatheter implantation of a stented heart valve prosthesis, including a balloon sizing procedure.

Heart valves, such as the mitral, tricuspid, aortic, and pulmonary valves, are sometimes damaged by disease or by aging, resulting in problems with the proper functioning of the valve. Heart valve problems generally take one of two forms: stenosis in which a valve does not open completely or the opening is too small, resulting in restricted blood flow; or insufficiency in which blood leaks backward across a valve when it should be closed.

Heart valve replacement has become a routine surgical procedure for patients suffering from valve regurgitation or stenotic calcification of the leaflets. Conventionally, the vast majority of valve replacements entail full stenotomy in placing the patient on cardiopulmonary bypass. Traditional open surgery inflicts significant patient trauma and discomfort, requires extensive recuperation times, and may result in life-threatening complications.

To address these concerns, within the last decade, efforts have been made to perform cardiac valve replacements using minimally-invasive techniques. In these methods, laparoscopic instruments are employed to make small openings through the patient's ribs to provide access to the heart. While considerable effort has been devoted to such techniques, widespread acceptance has been limited by the clinician's ability to access only certain regions of the heart using laparoscopic instruments.

Still other efforts have been focused upon percutaneous transcatheter (or transluminal) delivery of replacement cardiac valves to solve the problems presented by traditional open surgery and minimally-invasive surgical methods. In such methods, a valve prosthesis is compacted for delivery in a catheter and then advanced, for example through an opening in the femoral artery and through the descending aorta to the heart, where the prosthesis is then deployed in the valve annulus (e.g., the aortic valve annulus).

Various types and configurations of prosthetic heart valves are used in percutaneous valve procedures to replace diseased natural human heart valves. The actual shape and configuration of any particular prosthetic heart valve is dependent to some extent upon the valve being replaced (i.e., mitral valve, tricuspid valve, aortic valve, or pulmonary valve). In general, prosthetic heart valve designs attempt to replicate the function of the valve being replaced and thus will include valve leaflet-like structures used with either bioprostheses or mechanical heart valve prostheses. If bioprostheses are selected, the replacement valves may include a valved vein segment or pericardial manufactured tissue valve that is mounted in some manner within an expandable stent frame to make a valved stent. In order to prepare such a valve for percutaneous implantation, one type of valved stent can be initially provided in an expanded or uncrimped condition, then crimped or compressed around a balloon portion of a catheter until it is close to the diameter of the catheter. In other percutaneous implantation systems, the stent frame of the valved stent can be made of a self-expanding material. With these systems, the valved stent is crimped down to a desired size and held in that compressed state with a sheath, for example. Retracting the sheath from this valved stent allows the stent to expand to a larger diameter, such as when the valved stent is in a desired position within a patient. With either of these types of percutaneous stent delivery systems, conventional sewing of the prosthetic heart valve to the patient's native tissue is typically not necessary.

Adverse effects can occur if an implanted transcatheter heart valve does not fit appropriately, including paravalvular leakage and damage to surrounding tissues (e.g., aorta, cardiac tissue). However, there are significant limitations associated with current imaging-based sizing procedures for transcatheter heart valves. Limitations associated with imaging-based sizing procedures include, for example, the following: (1) Measurements are only taken in two dimensions, and therefore do not account for annular ellipticity; (2) identifying the true leaflet basal hinge point can be difficult with calcification, imaging errors, and the non-orthogonal geometry of a tricuspid valve; (3) unknown annular compliance makes cross-sectional geometry of an implanted frame difficult to predict, which can lead to unacceptable stent aspect ratios and valve performance; and (4) variable calcification profiles may interact unpredictably with the frame.

WO 2008/011261 A2 describes a system for deploying balloon-expandable heart valve. US 2008/009746 A1 describes assessment of aortic heart valve to facilitate repair or replacement. US 2004/093060 A1 describes a prosthetic valve for transluminal delivery.

In light of the above, a need exists for a catheter-based heart valve therapy system that can make a more accurate size determination, and deliver an appropriately sized heart valve prosthesis to the heart valve.

### Summary

One embodiment is directed to a catheter-based heart valve therapy system, which includes a stented heart valve prosthesis including a stent frame having a valvular contact region that is configured to make contact with a heart valve and nearby anatomy of a patient. The system includes a sizing balloon including a first region having a shape in an inflated state that matches a shape of the valvular contact region of the stent frame in an expanded state. The system also includes at least one catheter-based delivery system configured to deliver the sizing balloon to the heart valve of the patient for a size determination, and deliver the prosthesis to the heart valve for implantation.

Another embodiment is directed to a method of performing a therapeutic procedure on a heart valve, which includes providing a plurality of differently-sized stented heart valve prostheses. Each of the prostheses includes a stent frame with a valvular contact region that is configured to make contact with a heart valve and nearby anatomy of a patient. The valvular contact regions of the plurality of prostheses have a common shape in an expanded state. The method includes inflating a first sizing balloon within the heart valve of the patient to determine a size of the heart valve, wherein the inflating of the first sizing balloon causes a first portion of the first sizing balloon to assume a shape that matches the common shape. One of the stented heart valve prostheses is selected based on the determined size of the heart valve. The selected stented heart valve prosthesis is delivered to the heart valve via a catheter-based delivery system, and the selected stented heart valve prosthesis is deployed from the delivery system at the heart valve.

Yet another embodiment is directed to a catheter-based heart valve therapy system, which includes a plurality of differently-sized stented heart valve prostheses. Each of the prostheses includes a stent frame with a valvular contact region that is configured to make contact with a heart valve and nearby anatomy of a patient. The valvular contact regions of the plurality of prostheses have a common shape in an expanded state. The system includes a prosthesis-specific sizing balloon that corresponds to a prosthesis type of the plurality of prostheses and corresponds to a prosthesis size of a first subset of the plurality of prostheses. The sizing balloon includes a first region having a shape in an inflated state that is the same as the common shape. The system includes at least one catheter-based delivery system configured to deliver the sizing balloon to the heart valve of the patient for a size determination, and deliver one of the prostheses from the first subset to the heart valve for implantation.

### Brief Description of the Drawings

Figures 1A-1C are diagrams illustrating a system for delivering a transcatheter prosthetic heart valve to an implantation site according to one embodiment.
Figures 2A-2C are diagrams illustrating one embodiment of the stented heart valve prosthesis shown in Figures 1B and 1C.
Figure 3 is a diagram illustrating a prosthesis-specific sizing balloon corresponding to the stented heart valve prosthesis shown in Figures 2A-2C according to one embodiment.
Figures 4A-4C are diagrams illustrating another embodiment of the stented heart valve prosthesis shown in Figures 1B and 1C.
Figure 5 is a diagram illustrating a prosthesis-specific sizing balloon corresponding to the stented heart valve prosthesis shown in Figures 4A-4C according to one embodiment.
Figure 6 is a block diagram illustrating a catheter-based heart valve therapy system according to one embodiment.
Figure 7 is a flow diagram illustrating a method of performing a therapeutic procedure on a heart valve according to one embodiment.

### Detailed Description

The terms "distal" and "proximal" are used herein with reference to the treating clinician during the use of the catheter system; "Distal" indicates an apparatus portion distant from, or a direction away from the clinician and "proximal" indicates an apparatus portion near to, or a direction towards the clinician. The term "therapy" or "therapeutic procedure" as used herein in the context of heart valves is intended to include the repair of a heart valve, the replacement of a heart valve, or a combination of repair and replacement of a heart valve. While some of the description herein may refer specifically to therapy of aortic valves, the systems and methods disclosed herein can also generally be used for therapy of native or bioprosthetic mitral, pulmonic, or tricuspid valves.

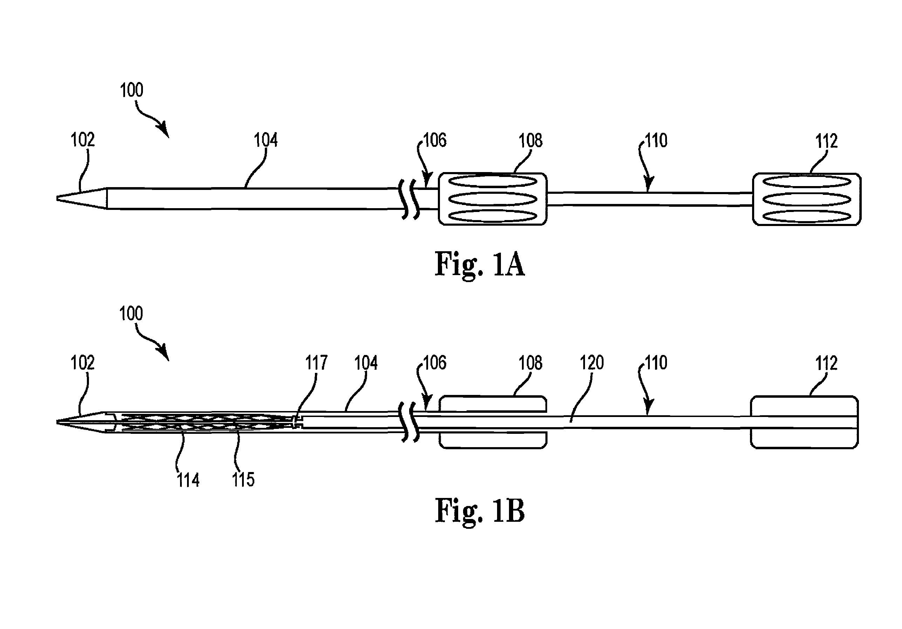

Figures 1A-1C are diagrams illustrating a system 100 for delivering a transcatheter prosthetic heart valve to an implantation site according to one embodiment. In the illustrated embodiment, the system 100 includes a shaft assembly 110 and a sheath assembly 106. The shaft assembly 110 includes a carrier shaft 120, a connector shaft 115, a nose cone 102, and a handle device 112. The connector shaft 115 interconnects the carrier shaft 120 and the nose cone 102, and in some constructions has a reduced-sized diameter to permit placement of the stented heart valve prosthesis 114 over the connector shaft 115. Though not shown in Figures 1A-1C, a guide wire lumen can be formed through the shafts 115 and 120.

Carrier shaft 120 is sized to be slidably received within a portion of the sheath assembly 106, and is configured in the illustrated embodiment for releasable coupling with the stented heart valve prosthesis 114. The carrier shaft 120 forms or includes a coupling device 117. The coupling device 117 is configured to selectively retain a proximal portion of the stented heart valve prosthesis 114. The coupling device 117 is configured to releasably mount the stented heart valve prosthesis 114 to the shaft assembly 110 when the prosthesis 114 is forced to a collapsed state within the sheath assembly 106. In this collapsed state, then, the stented heart valve prosthesis 114 will longitudinally move with movement of the shaft assembly 110. The sheath assembly 106 is configured to permit deployment of the stented heart valve prosthesis 114 from the loaded state shown in Figures 1A and 1B.

The nose cone 102 can assume a variety of forms, and is generally constructed to facilitate atraumatic placement of the delivery system 100 through a patient's vasculature and heart. The handle device 112 is mounted or connected to a proximal end of the carrier shaft 120, and provides a convenient surface for grasping by a clinician.

The sheath assembly 106 generally includes a sheath 104 and a handle device 108. The sheath 104 can be of a conventional catheter-like configuration (e.g., biocompatible polymer with or without an encapsulated wire braiding). In some constructions, the sheath 104 can further incorporate various steering features. Regardless, the sheath 104 is generally compliant, and is able to traverse the tortuous pathways associated with transcatheter heart valve implantation. The handle device 108 can assume a wide variety of forms, and is generally mounted or connected to a proximal end of the sheath 104. The sheath 104 defines a lumen sized to slidably receive the carrier shaft 120, as well as the stented heart valve prosthesis 114 in the collapsed state.

The delivery system 100 is operable to deliver or implant the stented heart valve prosthesis 114 as described in further detail below. Figures 1A and 1B illustrate the system 100 loaded with the stented heart valve prosthesis 114 prior to deployment. In particular, the stented heart valve prosthesis 114 is connected to the carrier shaft 120, for example via the coupling device 117, and is radially constrained within the sheath 104.

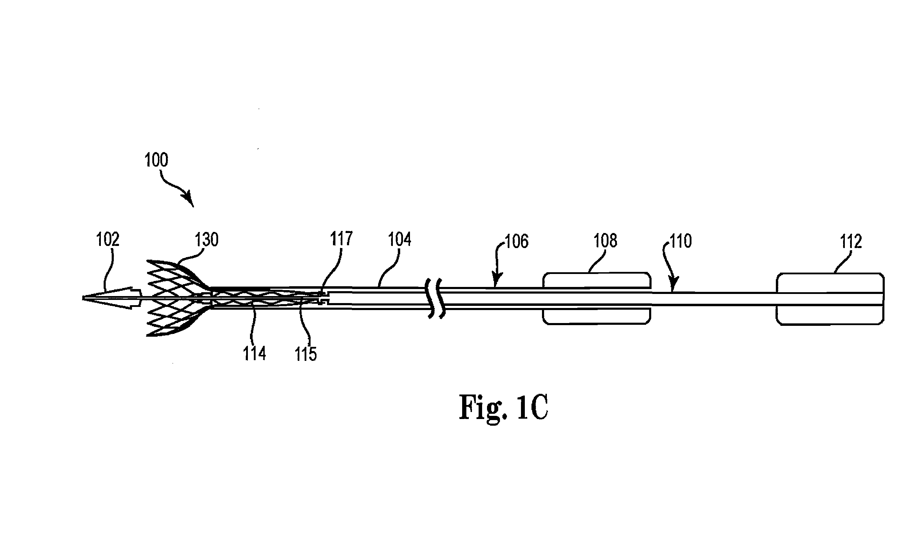

The loaded delivery system 100 is then advanced toward the implantation target site, for example in a retrograde manner through a cut-down to the femoral artery and into the patient's descending aorta. The delivery system 100 is then advanced, under fluoroscopic guidance, over the aortic arch, through the ascending aorta, and midway across the defective aortic valve (for aortic replacement). After positioning of the delivery system 100, the sheath 104 is partially retracted relative to the prosthesis 114 as shown in Figure 1C. For example, the handle device 108 provided with the sheath assembly 106 is retracted toward the handle device 112 of the shaft assembly 110. As shown, a distal region 130 of the prosthesis 114 is thus exteriorly exposed relative to the sheath 104, and begins to self-deploy. This proximal retraction of the sheath 104 continues, with a continually increasing length of the prosthetic 114 being exposed and thus partially deployed, until the prosthetic 114 is fully deployed at the native heart valve.

The delivery system 100 is useful with a variety of different configurations of a stented heart valve prostheses. In general terms, the prosthetic heart valve 114 includes a stent frame maintaining a valve structure (tissue or synthetic), with the stent frame having a normal, expanded state and collapsible to a collapsed state for loading within the system 100. The stent frame can be constructed to self-deploy or expand when released from the delivery system 100, or a separate expansion member can be provided (e.g., an expansion balloon). For example, the stented heart valve prosthesis 114 can be a prosthetic sold under the trade name CoreValve® available from Medtronic CoreValve, LLC. Other non-limiting examples of transcatheter heart valve prostheses useful with the system 100 are described in U.S. Publication Nos. 2006/0265056; 2007/0239266; and 2007/0239269;

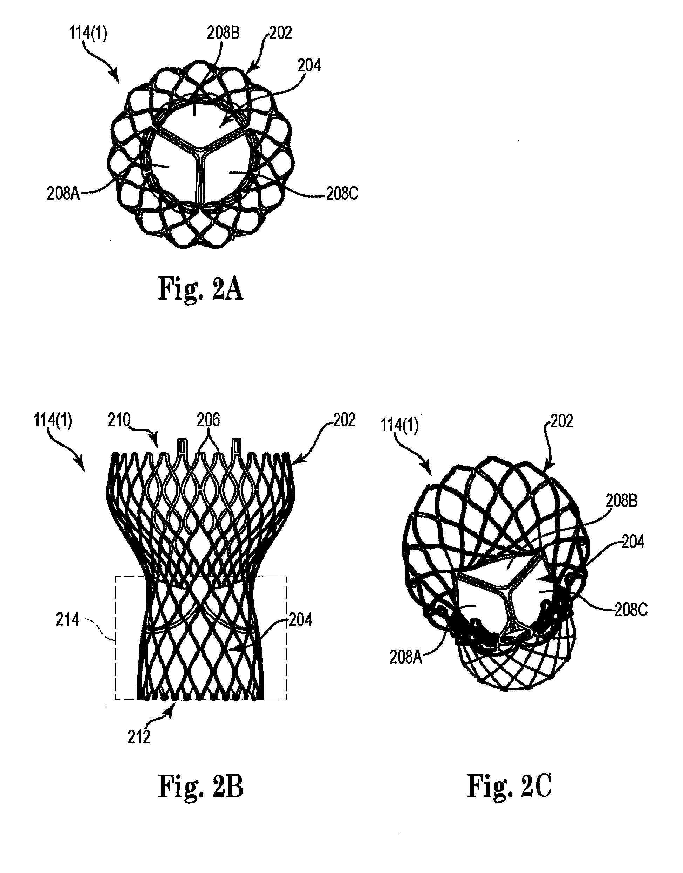

Figure 2A is a diagram illustrating a top view of one embodiment of the stented heart valve prosthesis 114 shown in Figures 1B and 1C. Figure 2B is a diagram illustrating a side view of the stented heart valve prosthesis 114 shown in Figure 2A according to one embodiment. Figure 2C is a diagram illustrating a perspective view of the stented heart valve prosthesis 114 shown in Figure 2A according to one embodiment. The embodiment of the stented heart valve prosthesis 114 shown in Figures 2A-2C is identified by reference number 114(1). Prosthesis 114(1) is compressible to a relatively small diameter for percutaneous delivery to the heart of a patient, and is then self-expandable via removal of external compressive forces.

As shown in Figures 2A-2C, stented heart valve prosthesis 114(1) includes a stent frame 202 and a valve structure 204. The stent frame 202 is a self-expanding support structure that includes a number of strut or wire portions 206 arranged relative to each other to provide a desired compressibility and strength to the prosthesis 114(1). Stent frame 202 includes a valvular contact region 214 that is configured to make contact with a heart valve and nearby anatomy of a patient. Stent frame 202 can be made from a shape memory material, such as Nitinol. Valve structure 204 is mounted inside of the stent frame 202, and includes a plurality of leaflets 208A-208C (collectively referred to as leaflets 208). In the illustrated embodiment, valve structure 204 includes three leaflets 208. In other embodiments, valve structure 204 may include more or less than three leaflets 208. Figure 2B also shows a proximal outflow end 210 and a distal inflow end 212 of prosthesis 114(1). Valvular contact region 214 in the illustrated embodiment extends from the bottom end of the valve structure 204 (adjacent to the distal inflow end 212 of the stent frame 202) to the top end of the valve structure 204 (which corresponds to the position of the sinotubular junction).

As discussed above in the Background section, it is important for stented heart valve prostheses to be sized appropriately to help ensure a proper fit with the patient's anatomy, but there are several limitations associated with current imaging-based sizing procedures. One embodiment provides a heart valve therapy system that uses prosthesis-specific sizing balloons to facilitate identification of an appropriately sized heart valve prosthesis. Different types of stented heart valve prostheses typically have different shapes, and each type typically includes multiple sizes. In one embodiment, a differently-shaped balloon is used for each different type of prosthesis, and a different sized balloon is used for each different prosthesis size. The specific shape of the sizing balloons varies based on the prosthesis shape. In one embodiment, the sizing balloons disclosed herein are configured to be delivered, inflated, and deflated, using a conventional catheter-based delivery system.

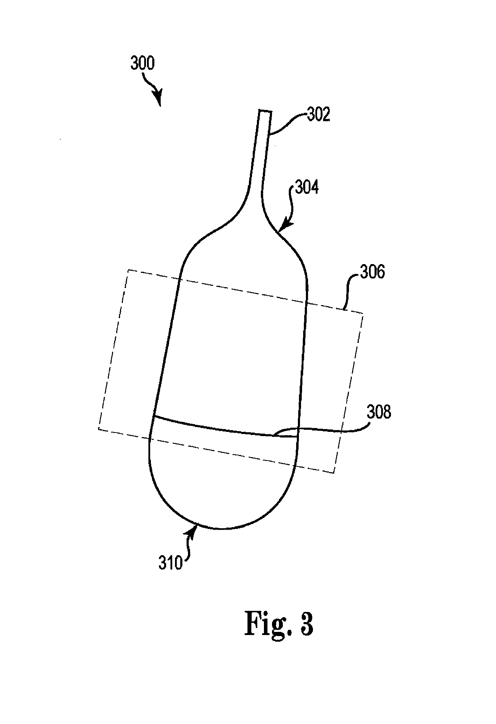

Figure 3 is a diagram illustrating a prosthesis-specific sizing balloon 300 corresponding to the stented heart valve prosthesis 114(1) shown in Figures 2A-2C according to one embodiment. Sizing balloon 300 includes a tube 302, a proximal end 304, a valvular contact region 306, a visually detectable marker 308, and a distal end 310. Tube 302 is configured to be fluidly connected to an inflation/deflation source (not shown) for inflating and deflating the sizing balloon 300. Valvular contact region 306 is configured to make contact with a heart valve of a patient when sizing balloon 300 is in an expanded state. Sizing balloon 300 according to one embodiment is a non-compliant balloon that is configured to be used to estimate the size of the aortic annulus in a patient to facilitate the identification of an appropriately sized heart valve prosthesis.

In one embodiment, valvular contact region 306 of balloon 300 has an inflated shape and size that matches (e.g., is identical to or substantially identical to) the shape and size of the valvular contact region 214 (Figure 2B) of stented heart valve prosthesis 114(1) in the expanded state. In the expanded state, both regions 214 and 306 are gradually tapered such that the diameter of these regions gradually increases going from the proximal end to the distal end. Thus, the diameter of these regions is larger at the distal end (e.g., flared at the inflow end) than at the proximal end. This taper helps keep the balloon 300 in place during the sizing procedure.

The use of a balloon 300 with a valvular contact region 306 that matches the shape and size of the valvular contact region 214 of the prosthesis 114(1) provides numerous benefits. For example, anterior mitral leaflet and coronary ostia interactions can be predicted for the prosthesis 114(1) by monitoring these interactions during use of the balloon 300. As another example, balloon 300 according to one embodiment has a compliance or radial flexibility that is the same as or substantially the same as that of the stent frame 202, and the balloon 300 is configured to generate the same or substantially the same radial force as the stent frame 202. This allows the balloon 300 to simulate the radial force of the stent frame 202 on the native anatomy and predict how the frame 202 would interact with and be deformed by the native anatomy, such as predicting paravalvular leakage, heart block, and post-installation aspect ratio of the stent frame 202. For example, if the balloon sizing procedure indicated that the stent frame 202 would be deformed to an unacceptable degree by the native anatomy, a different size and/or type of stent frame may be chosen for the procedure. The use of sizing balloon 300 also allows the size of the perimeter of the valve annulus to be determined, as opposed to just a diameter measurement (which may not be an accurate indicator of size if the valve annulus is highly elliptical or irregular).

In one embodiment, a conventional imaging technique (e.g., CT, MRI, echocardiography) is used to make an initial estimate of the size of the native heart valve, and a sizing balloon 300 with a size corresponding to the initial estimate is chosen for the sizing procedure. After inflation of this sizing balloon 300 within the native heart valve of a patient, a conventional imaging technique is employed to assess the fit of the expanded balloon 300 within the heart valve. After a determination is made regarding the fit of the expanded balloon 300 within the heart valve, an appropriately sized version of the stented heart valve prosthesis 114(1) is selected based on this determination. If the expanded balloon 300 is substantially over-sized or under-sized, a differently-sized balloon 300 may be selected and the sizing procedure repeated.

In another embodiment, rather than using imaging techniques, or in addition to using imaging techniques, the fit of the expanded balloon 300 within the heart valve is assessed using intraballoon pressure information. In one form of this embodiment, prior to delivery of the balloon 300 inside the patient, the balloon 300 is inflated to a predetermined intraballoon pressure and the diameter of the inflated balloon 300 is measured along with the volume of liquid injected into the balloon 300. After insertion of the balloon 300 into the heart valve, the balloon 300 is again inflated using the same volume of fluid, and the intraballoon pressure is measured again. A measured pressure that is greater than the predetermined pressure indicates that the native heart valve is exerting force on the balloon 300, and the balloon 300 may be of the correct size. A measured pressure that is the same as the predetermined pressure indicates that the native heart valve is not exerting force on the balloon 300, and the balloon 300 may be too small.

In one embodiment, the visually detectable marker 308 on the sizing balloon 300 is configured to be aligned with the heart valve annulus (e.g., aortic basal annulus) of the native heart valve. The visually detectable marker 308 on the balloon 300 facilitates accurate placement of the balloon 300 within the native valve. In the illustrated embodiment, marker 308 is a circular line drawn around the circumference of the balloon 300.

In one embodiment, in addition to being used for sizing purposes, balloon 300 is also configured to be used for balloon aortic valvuloplasty (BAV) for relief of aortic valvular stenosis. In one form of this embodiment, a BAV procedure is performed using balloon 300 by applying a high radial force to break open fused commissures in the native valve, and then a reduced radial force is applied to approximate the radial force of the stent frame to assess sizing. In one embodiment, the BAV procedure is performed using a single balloon. In another embodiment, the BAV procedure is performed using a balloon-in-balloon system. In one form of this embodiment, balloon 300 is configured as a balloon-in-balloon system including an inner balloon and an outer balloon with different radial forces (e.g., a higher radial force outer balloon and a lower radial force inner balloon, or vice versa).

As mentioned above, in one embodiment, a differently-shaped balloon 300 is used for each different type of prosthesis 114 (with a balloon shape that corresponds to the shape of the particular type of prosthesis being used), and a different sized balloon is used for each different prosthesis size. In one embodiment, each balloon 300 and each prosthesis 114 correspond to a single size (e.g., 23, 26, 29, or 31mm). In another embodiment, each balloon 300 corresponds to and is inflatable to a range of sizes (e.g., one balloon 300 with a range of 20-24mm, another balloon 300 with a range of 25-29mm, and another balloon 300 with a range of 30-34mm). Thus, for example, in one form of this embodiment, if the native valve had an actual size of 23mm, and a balloon 300 with a range of 20-24mm was being used, the balloon 300 would gradually be expanded to 20mm, 21mm, 22mm, etc., as it was being monitored with a conventional imaging technique. Interference may begin to be seen when the balloon 300 was expanded to about 23mm, and the shape of the balloon 300 may begin to be deformed as it is expanded to 24mm. Thus, the sizing procedure may result in a determination that the native valve size is between about 23mm and 24mm.

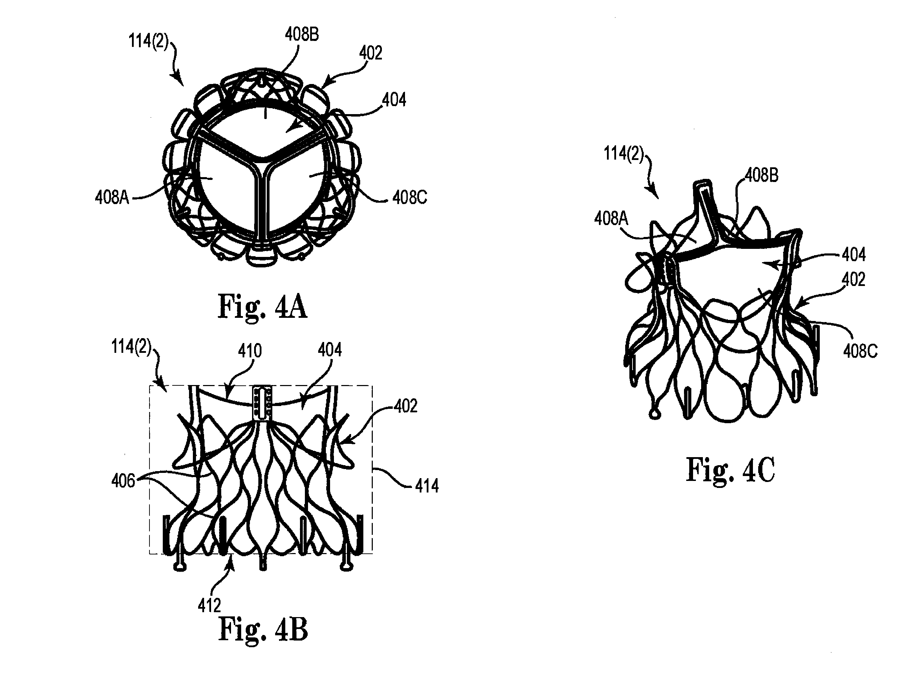

Figure 4A is a diagram illustrating a top view of another embodiment of the stented heart valve prosthesis 114 shown in Figures 1B and 1C. Figure 4B is a diagram illustrating a side view of the stented heart valve prosthesis 114 shown in Figure 4A according to one embodiment. Figure 4C is a diagram illustrating a perspective view of the stented heart valve prosthesis 114 shown in Figure 4A according to one embodiment. The embodiment of the stented heart valve prosthesis 114 shown in Figures 4A-4C is identified by reference number 114(2). Prosthesis 114(2) is compressible to a relatively small diameter for percutaneous delivery to the heart of a patient, and is then self-expandable via removal of external compressive forces.

As shown in Figures 4A-4C, stented heart valve prosthesis 114(2) includes a stent frame 402 and a valve structure 404. The stent frame 402 is a self-expanding support structure that includes a number of strut or wire portions 406 arranged relative to each other to provide a desired compressibility and strength to the prosthesis 114(2). Stent frame 402 includes a valvular contact region 414 that is configured to make contact with a heart valve and nearby anatomy of a patient. Stent frame 402 can be made from a shape memory material, such as Nitinol. Valve structure 404 is mounted inside of the stent frame 402, and includes a plurality of leaflets 408A-408C (collectively referred to as leaflets 408). In the illustrated embodiment, valve structure 404 includes three leaflets 408. In other embodiments, valve structure 404 may include more or less than three leaflets 408. Figure 4B also shows a proximal outflow end 410 and a distal inflow end 412 of prosthesis 114(2). Valvular contact region 414 according to the illustrated embodiment extends from the bottom end of the valve structure 404 (adjacent to the distal inflow end 412 of the stent frame 402) to the top end of the valve structure 404 (which corresponds to the position of the sinotubular junction).

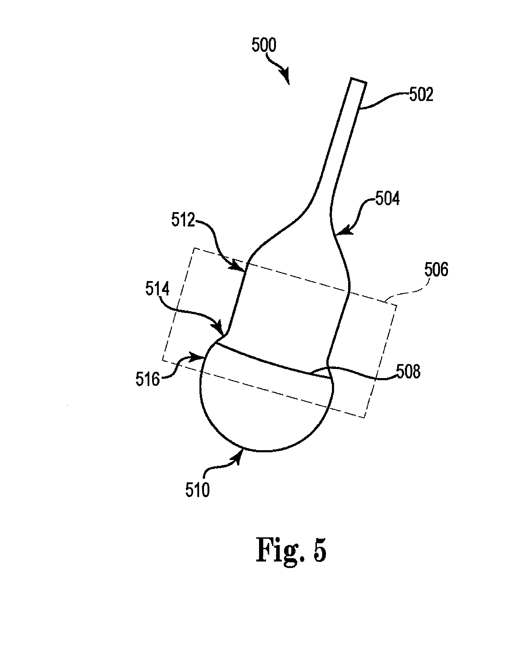

Figure 5 is a diagram illustrating a prosthesis-specific sizing balloon 500 corresponding to the stented heart valve prosthesis 114(2) shown in Figures 4A-4C according to one embodiment. Sizing balloon 500 includes a tube 502, a proximal end 504, a valvular contact region 506, a visually detectable marker 508, and a distal end 510. Tube 502 is configured to be fluidly connected to an inflation/deflation source (not shown) for inflating and deflating the sizing balloon 500. Valvular contact region 506 is configured to make contact with a heart valve of a patient when sizing balloon 500 is in an expanded state. Sizing balloon 500 according to one embodiment is a non-compliant balloon that is configured to be used to estimate the size of the aortic annulus in a patient to facilitate the identification of an appropriately sized heart valve prosthesis.

In one embodiment, valvular contact region 506 of balloon 500 has an inflated shape and size that matches (e.g., is identical to or substantially identical to) the shape and size of the valvular contact region 414 (Figure 4B) of stented heart valve prosthesis 114(2) in the expanded state. In the expanded state, both regions 414 and 506 have a first portion (indicated at 512 in Figure 5) with a constant or substantially constant diameter and a second portion (indicated at 516) with a different constant or substantially constant diameter, which are connected by a tapered portion (indicated at 514) with a varying diameter. As shown in Figure 5, the second portion 516 has a larger diameter than the first portion 512, and the tapered portion 514 is at an angle of about forty-five degrees. The diameter of the regions 414 and 506 is larger at the distal end (e.g., flared at the inflow end) than at the proximal end, which helps keep the balloon 500 in place during the sizing procedure. In one embodiment, balloon 500 is configured to be used in the same manner as described above with respect to balloon 300.

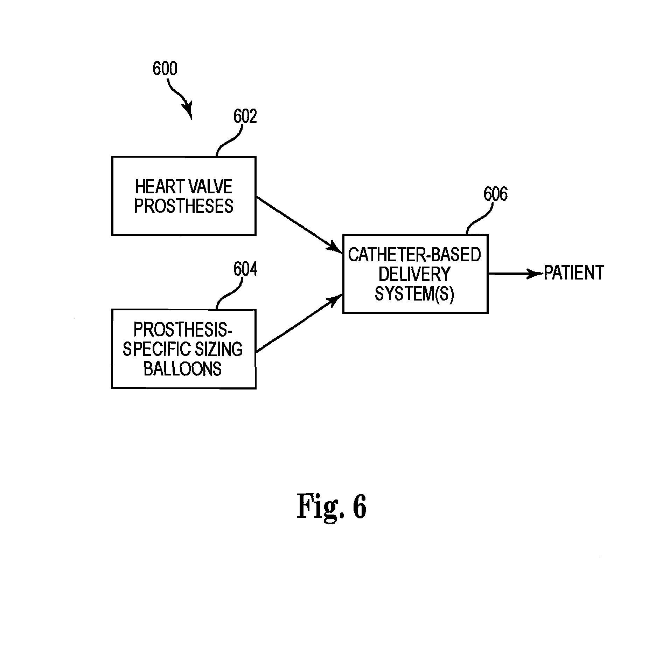

Figure 6 is a block diagram illustrating a catheter-based heart valve therapy system 600 according to one embodiment. System 600 includes a plurality of heart valve prostheses 602, a plurality of prosthesis-specific sizing balloons 604, and at least one catheter-based delivery system 606. In one embodiment, the prostheses 602 comprise a plurality of differently-sized stented heart valve prostheses, and each of the prostheses 602 includes a stent frame having a valvular contact region that is configured to make contact with a heart valve and nearby anatomy of a patient. The valvular contact regions of the plurality of prostheses 602 according to one embodiment have a common shape in an expanded state. The common shape in one embodiment has a varying diameter. In one embodiment, the prosthesis-specific sizing balloons 604 each include a first region having a shape in an inflated state that matches the common shape. In one embodiment, each prosthesis-specific sizing balloon 604 corresponds to a prosthesis type of the prostheses 602 and corresponds to a prosthesis size of a subset of the prostheses 602. The at least one catheter-based delivery system 606 is configured to deliver one or more of the sizing balloons 604 to the heart valve of a patient for a size determination, and deliver one of the prostheses 602 to the heart valve of the patient for implantation.

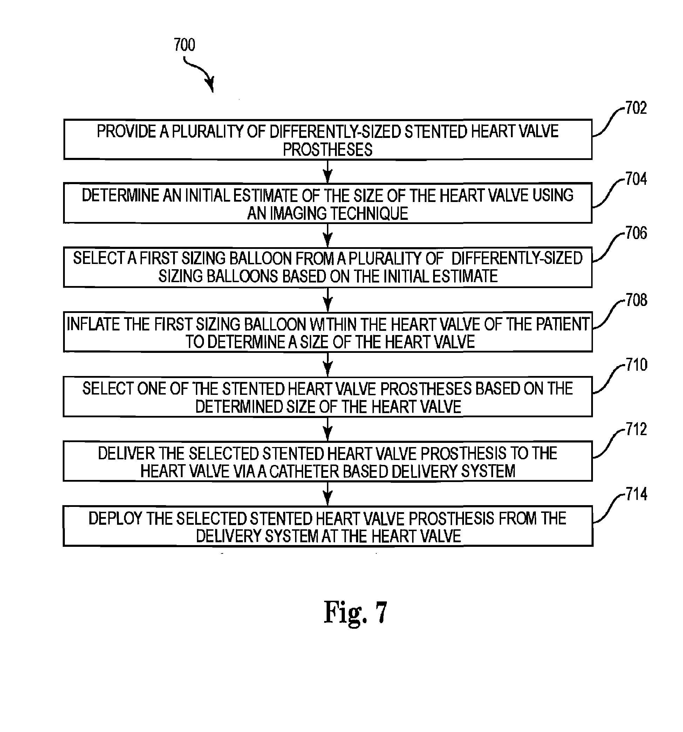

Figure 7 is a flow diagram illustrating a method 700 of performing a therapeutic procedure on a heart valve according to one embodiment. At 702, a plurality of differently-sized stented heart valve prostheses are provided. In one embodiment, each of the prostheses provided at 702 includes a stent frame with a valvular contact region that is configured to make contact with a heart valve and nearby anatomy of a patient. The valvular contact regions of the plurality of prostheses have a common shape in an expanded state in one embodiment. At 704, an initial estimate of the size of the heart valve is determined using an imaging technique. At 706, a first sizing balloon is selected from a plurality of differently-sized sizing balloons based on the initial estimate. At 708, the first sizing balloon is inflated within the heart valve of the patient to determine a size of the heart valve. In one embodiment, the inflating of the first sizing balloon at 708 causes a first portion of the first sizing balloon to assume a shape that matches the common shape. In one embodiment, the size of the heart valve is determined at 708 based on imaging the inflating of the first sizing balloon. In another embodiment, the size of the heart valve is determined at 708 based additionally on intraballoon pressure information. At 710, one of the stented heart valve prostheses is selected based on the determined size of the heart valve. At 712, the selected stented heart valve prosthesis is delivered to the heart valve via a catheter-based delivery system. At 714, the selected stented heart valve prosthesis is deployed from the delivery system at the heart valve. In one embodiment of method 700, a balloon aortic valvuloplasty (BAV) procedure is performed with the first sizing balloon prior to determining the size of the heart valve.

The sizing balloon techniques help provide an optimized fit of the implanted heart valve prosthesis, thereby reducing device-related complications associated with inappropriate sizing. Although the systems and methods disclosed herein have been primarily described in the context of stented heart valve prostheses, it will be understood that embodiments of the balloon sizing techniques may also be applied to other devices and other portions of a patient's anatomy, including sizing catheter-delivered annuloplasty rings/bands.

## Claims

1. A catheter-based heart valve therapy system (100), comprising:
a stented heart valve prosthesis (114) including a stent frame (202) having a valvular contact region (214) that is configured to make contact with a heart valve and nearby anatomy of a patient;
a sizing balloon (300; 604) including a first region (306) having a shape in an inflated state that matches a shape of the valvular contact region (214) of the stent frame (202) in an expanded state; and
at least one catheter-based delivery system (606) configured to deliver the sizing balloon (300; 604) to the heart valve of the patient for a size determination, and deliver the prosthesis (114) to the heart valve for implantation.

2. The heart valve therapy system of claim 1, wherein the sizing balloon (300; 604) is a prosthesis-specific sizing balloon that corresponds to a prosthesis type and a prosthesis size of the stented heart valve prosthesis (114).

3. The heart valve therapy system of claim 1, wherein the valvular contact region (214) of the prosthesis (114) and the first region (306) of the sizing balloon (300; 604) each have a shape that is tapered such that a diameter of these regions increases going from a proximal end of these regions to a distal end of these regions.

4. The heart valve therapy system of claim 3, wherein the distal end of the prosthesis (114) corresponds to an inflow end of the prosthesis (114).

5. The heart valve therapy system of claim 1, wherein the valvular contact region (214) of the prosthesis (114) and the first region (306) of the sizing balloon (300; 604) each have a shape that includes a first portion (512) with a first substantially constant diameter and a second portion (516) with a second substantially constant diameter, wherein the first and second diameters are different.

6. The heart valve therapy system of claim 5, wherein the valvular contact region (214) of the prosthesis (114) and the first region (306) of the sizing balloon (300; 604) each have a shape that further includes a tapered portion with a varying diameter that connects the first portion and the second portion.

7. The heart valve therapy system of claim 5, wherein the second portion corresponds to distal ends of the prosthesis (114) and balloon (300; 604), and wherein the second substantially constant diameter is larger than the first substantially constant diameter.

8. The heart valve therapy system of claim 1, wherein the sizing balloon (300; 604) is configured to generate a radial force on the heart valve that is substantially similar to an amount of radial force that is generated by the prosthesis (114).

9. The heart valve therapy system of claim 1, wherein the sizing balloon (300; 604) has a compliance that is substantially similar to a compliance of the stent frame (202).

10. The heart valve therapy system of claim 1, wherein the sizing balloon (300; 604) includes a visually detectable marker (308; 508) that is configured to facilitate accurate placement of the balloon (300; 604) within the heart valve.

11. The heart valve therapy system of claim 10, wherein the visually detectable marker (308; 508) is configured to be aligned with an annulus of the heart valve.

## Patentansprüche

1. Katheterbasiertes Herzklappentherapiesystem (100), umfassend:
eine Herzklappenprothese mit Stent (114), aufweisend einen Stentrahmen (202) mit einer Klappenkontaktregion (214), die dazu konfiguriert ist, mit einer Herzklappe und umliegender Anatomie eines Patienten in Kontakt zu treten;
einen Größenbestimmungsballon (300; 604), aufweisend eine erste Region (306) mit einer Form in einem aufgeblasenen Zustand, die mit einer Form der Klappenkontaktregion (214) des Stentrahmens (202) in einem ausgedehnten Zustand übereinstimmt; und
wenigstens ein katheterbasiertes Abgabesystem (606), das dazu konfiguriert ist, den Größenbestimmungsballon (300; 604) zur Größenbestimmung an die Herzklappe des Patienten abzugeben und die Prothese (114) zur Implantation an die Herzklappe abzugeben.

2. Herzklappentherapiesystem nach Anspruch 1, wobei der Größenbestimmungsballon (300; 604) ein prothesenspezifischer Größenbestimmungsballon ist, der einem Prothesentyp und einer Prothesengröße der Herzklappenprothese mit Stent (114) entspucht.

3. Herzklappentherapiesystem nach Anspruch 1, wobei die Klappenkontaktregion (214) der Prothese (114) und die erste Region (306) des Größenbestimmungsballons (300; 604) jeweils eine Form aufweisen, die derart verjüngt ist, dass ein Durchmesser dieser Regionen von einem proximalen Ende dieser Region zu einem distalen Ende dieser Regionen hin zunimmt.

4. Herzklappentherapiesystem nach Anspruch 3, wobei das distale Ende der Prothese (114) einem Einlaufende der Prothese (114) entspricht.

5. Herzklappentherapiesystem nach Anspruch 1, wobei die Klappenkontaktregion (214) der Prothese (114) und die erste Region (306) des Größenbestimmungsballons (300; 604) jeweils eine Form aufweisen, die einen ersten Abschnitt (512) mit einem ersten im Wesentlichen konstanten Durchmesser und einen zweiten Abschnitt (516) mit einem zweiten im Wesentlichen konstanten Durchmesser aufweist, wobei der erste und zweite Durchmesser unterschiedlich sind.

6. Herzklappentherapiesystem nach Anspruch 5, wobei die Klappenkontaktregion (214) der Prothese (114) und die erste Region (306) des Größenbestimmungsballons (300; 604) jeweils eine Form aufweisen, die ferner einen verjüngten Abschnitt mit einem variierenden Durchmesser aufweist, der den ersten und zweiten Abschnitt verbindet.

7. Herzklappentherapiesystem nach Anspruch 5, wobei der zweite Abschnitt distalen Enden der Prothese (114) und des Ballons (300; 604) entspricht, und wobei der zweite im Wesentlichen konstante Durchmesser größer als der erste im Wesentlichen konstante Durchmesser ist.

8. Herzklappentherapiesystem nach Anspruch 1, wobei der Größenbestimmungsballon (300; 604) dazu konfiguriert ist, eine radiale Kraft an der Herzklappe zu erzeugen, die im Wesentlichen ähnlich einer Stärke radialer Kraft ist, die durch die Prothese (114) erzeugt wird.

9. Herzklappentherapiesystem nach Anspruch 1, wobei der Größenbestimmungsballon (300; 604) eine elastische Nachgiebigkeit aufweist, die im Wesentlichen ähnlich einer elastischen Nachgiebigkeit des Stentrahmens (202) ist.

10. Herzklappentherapiesystem nach Anspruch 1, wobei der Größenbestimmungsballon (300; 604) eine optisch erkennbare Markierung (308; 508) aufweist, die dazu konfiguriert ist, die genaue Anordnung des Ballons (300; 604) in der Herzklappe zu unterstützen.

11. Herzklappentherapiesystem nach Anspruch 10, wobei die optisch erkennbare Markierung (308; 508) dazu konfiguriert ist, an einem Anulus der Herzklappe ausgerichtet zu werden.

## Revendications

1. Système (100) de thérapie des valvules cardiaques basé sur un cathéter, comprenant :
une prothèse (114) de valvule cardiaque à stent comprenant une armature (202) de stent ayant une région (214) de contact valvulaire qui est configurée pour établir un contact avec une valvule cardiaque et l'anatomie voisine d'un patient :
un ballonnet (300 ; 604) de dimensionnement comprenant une première région (306) ayant une forme, à l'état gonflé, qui correspond à une forme de la région (214) de contact valvulaire de l'armature (202) de stent dans un état déployé ; et
au moins un système (606) de distribution basé sur un cathéter configuré pour poser le ballonnet (300 ; 604) de dimensionnement au niveau de la valvule cardiaque du patient pour une détermination de dimension, et la pose de la prothèse (114) au niveau de la valvule cardiaque pour une implantation.

2. Système de thérapie des valvules cardiaques selon la revendication 1, dans lequel le ballonnet (300 ; 604) de dimensionnement est un ballonnet de dimensionnement spécifique à la prothèse, correspondant à un type et une taille prothétiques de la prothèse (114) de valvule cardiaque à stent.

3. Système de thérapie des valvules cardiaques selon la revendication 1, dans lequel la région (214) de contact valvulaire de la prothèse (114) et la première région (306) du ballonnet (300 ; 604) de dimensionnement ont chacune une forme effilée de telle sorte qu'un diamètre de ces régions augmente à partir d'une extrémité proximale de ces régions vers une extrémité distale de ces régions.

4. Système de thérapie des valvules cardiaques selon la revendication 3, dans lequel l'extrémité distale de la prothèse (114) correspond à une extrémité d'entrée de la prothèse (114)

5. Système de thérapie des valvules cardiaques selon la revendication 1, dans lequel la région (214) de contact valvulaire de la prothèse (114) et la première région (306) du ballonnet (300 ; 604) de dimensionnement ont chacune une forme qui comprend une première partie (512) ayant un premier diamètre sensiblement constant et une deuxième partie (516) ayant un deuxième diamètre sensiblement constant, les premier et deuxième diamètres étant différents.

6. Système de thérapie des valvules cardiaques selon la revendication 5, dans lequel la région (214) de contact valvulaire de la prothèse (114) et la première région (306) du ballonnet (300 ; 604) de dimensionnement ont chacune une forme qui comprend en outre une partie effilée dont le diamètre varie, et qui raccorde la première partie et la deuxième partie.

7. Système de thérapie des valvules cardiaques selon la revendication 5, dans lequel la deuxième partie correspond à des extrémités distales de la prothèse (114) et du ballonnet (300 ; 604), et dans lequel le deuxième diamètre sensiblement constant est supérieur au premier diamètre sensiblement constant.

8. Système de thérapie des valvules cardiaques selon la revendication 1, dans lequel le ballonnet (300 ; 604) de dimensionnement est configuré pour générer une force radiale s'exerçant sur la valvule cardiaque, force qui est sensiblement analogue à une force radiale générée par la prothèse (114).

9. Système de thérapie des valvules cardiaques selon la revendication 1, dans lequel le ballonnet (300 ; 604) de dimensionnement a une élasticité analogue à l'élasticité de l'armature (202) de stent.

10. Système de thérapie des valvules cardiaques selon la revendication 1, dans lequel le ballonnet (300 ; 604) de dimensionnement comprend un marqueur (308 ; 508) détectable visuellement et qui est configuré pour faciliter le placement précis du ballonnet (300 ; 604) dans la valvule cardiaque.

11. Système de thérapie des valvules cardiaques selon la revendication 10, dans lequel le marqueur (308 ; 508) détectable visuellement est configuré pour être aligné avec un anneau de la valvule cardiaque.
